(19) Europäisches Patentamt
European Patent Office
Office européen des brevets

(11) Veröffentlichungsnummer: **0 421 125 A2**

# (12) EUROPÄISCHE PATENTANMELDUNG

(21) Anmeldenummer: **90116709.8**

(22) Anmeldetag: **31.08.90**

(51) Int. Cl.[5]: **G03B 27/34**, C07D 409/06, G03B 7/16, C07D 413/06, G03B 17/20, C07D 405/14, C07D 409/14, C07D 401/14, C07D 413/14, C07D 411/14, C07D 405/04, //C07D409/04, C07D413/04,C07D411/04, A01N43/653,A01N43/50, A01N43/72

(30) Priorität: **09.09.89 DE 3930166**
**21.12.89 DE 3942333**

(43) Veröffentlichungstag der Anmeldung:
**10.04.91 Patentblatt 91/15**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Goetz, Norbert, Dr.**
**Schoefferstrasse 25**
**W-6520 Worms 1(DE)**
Erfinder: **Kober, Reiner, Dr.**
**Im Schlittweg 20**
**W-6701 Fussgoenheim(DE)**
Erfinder: **Zipperer, Bernhard, Dr.**
**Am Herrgottsacker 6**
**W-6716 Dirmstein(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**
Erfinder: **Gebhardt, Joachim, Dr.**
**Chenoverstrasse 17**
**W-6703 Limburgerhof(DE)**

## (54) Fungizide Azolylmethyloxirane.

(57) Azolylmethyloxirane I

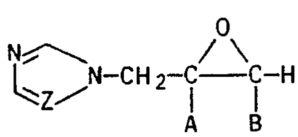

I

(A, B = 5-/6-gliedriges Heteroaryl, Alkyl, Cycloalkyl, Tetrahydropyranyl, Tetrahydrothiopyranyl, Phenyl, Biphenyl, Naphthyl oder Benzyl, wobei die Substituenten A und B noch einen bis drei weitere Reste tragen können, mit der Maßgabe, daß mindestens einer der Substituenten A oder B Heteroaryl bedeutet, es sei denn, B bedeutet eine o-Methylphenylgruppe; Z = CH, N)
sowie ihre Säureadditionssalze und Metallkomplexe,
ausgenommen 2-(Imidazol-1-ylmethyl)-2-(4-fluorphenyl)-3-(pyrid-3-yl)-oxiran.

Die Verbindungen I eignen sich als Fungizide.

EP 0 421 125 A2

Xerox Copy Centre

## FUNGIZIDE AZOLYLMETHYLOXIRANE

Die vorliegende Erfindung betrifft neue Azolylmethyloxirane der Formel I

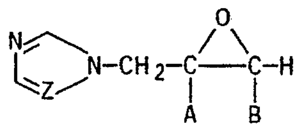

I,

in der die Substituenten die folgende Bedeutung haben:

A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl, wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,

mit der Maßgabe, daß mindestens einer der Substituenten A oder B eine der oben definierten Heteroarylgruppen bedeutet, es sei denn, B bedeutet eine o-Methylphenylgruppe;

Z CH oder N

sowie ihre pflanzenverträglichen Säureadditionssalze und Metallkomplexe, ausgenommen 2-(Imidazol-1-ylmethyl)-2-(4-fluorphenyl)-3-(pyrid-3-yl)-oxiran.

Außerdem betrifft die Erfindung Verfahren zur Herstellung dieser Verbindungen, ihre Verwendung als Fungizide und fungizide Mittel, welche diese Verbindungen als wirksame Substanzen enthalten.

Des weiteren betrifft die Erfindung Oxiran-Zwischenprodukte der Formel III

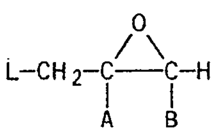

III,

wobei L Halogen oder einen Rest R-$SO_2$-O- und R $C_1$-$C_6$-Alkyl, partiell oder vollständig halogeniertes $C_1$-$C_4$-Alkyl oder Phenyl, das durch Halogen, $C_1$-$C_4$-Alkyl oder Nitro substituiert sein kann, bedeutet.

Es ist bekannt, cis-2-(1,2,4-Triazol-1-yl-methyl)-2-(4-chlorphenyl)-3-(4-tert.-butylphenyl)-oxiran (EP 94564) und cis-2-(1,2,4-Triazol-1-yl-methyl)-2-(4-methylphenyl)-3-(2-fluorphenyl)-oxiran (EP 196038) als Fungizide zu verwenden.

Des weiteren werden in der EP-A 332 073 Azolylmethyloxirane vom Typ der Verbindungen I erwähnt, die am Oxiranring in 2-Stellung eine Imidazolylmethyl- und eine 4-Fluorphenylgruppe und in 3-Stellung u.a. eine Pyridylgruppe tragen.

Die fungiziden Wirkungen dieser Verbindungen können jedoch, besonders bei kleinen Aufwandmengen und -konzentrationen, nur bedingt befriedigen.

Der Erfindung lag daher die Aufgabe zugrunde, neue fungizid wirksame Substanzen zu finden.

Demgemäß wurden die eingangs definierten Azolylmethyloxirane der Formel I gefunden. Weiterhin wurden Zwischenprodukte der Formel III zur Herstellung dieser Azolylmethyloxirane gefunden.

Im einzelnen haben die Substituenten in den erfindungsgemäßen Verbindungen I bzw. III die folgende Bedeutung:

A, B - 5- oder 6-gliedriges Heteroaryl, insbesondere Pyrrol-2-yl, Pyrrol-3-yl, Furan-2-yl, Thien-2-yl, Thien-3-yl, Pyrid-2-yl, Pyrid-3-yl, Pyrid-4-yl, 1,3-Diazol-2-yl, Oxazol-4-yl, Oxazol-5-yl, Isoxazol-3-yl, Isoxazol-4-yl, Isoxazol-5-yl, Thiazol-4-yl, Thiazol-5-yl, Imidazol-4-yl, 1,2,4-Triazol-3-yl und 1,3,4-Triazol-2-yl. Diese Substituenten können noch eine Nitro- oder Aminogruppe oder an jedem C-Atom noch einen der folgenden Reste tragen: Halogen wie Fluor, Chlor und Brom, $C_1$-$C_4$-Alkyl wie Methyl, Ethyl, n-Butyl und tert.-Butyl, partiell oder vollständig durch Fluor, Chlor und/oder Brom halogeniertes $C_1$-$C_4$-Alkyl wie Trifluormethyl, Pentafluorethyl und 2-Chlor-1,1,2-trifluorethyl, $C_1$-$C_4$-Alkoxy wie Methoxy, Ethoxy und tert.-Butoxy oder Phenoxy;

- verzweigtes oder unverzweigtes $C_1$-$C_8$-Alkyl, das eine Nitro-oder Aminogruppe oder bis zu drei der bei der Heteroarylgruppe genannten Reste tragen kann, bevorzugt $C_1$-$C_6$-Alkyl wie Methyl, Ethyl, n-Propyl, Isopropyl, n-Butyl, sec.-Butyl, tert.-Butyl, n-Pentyl und Neopentyl;
- $C_3$-$C_8$-Cycloalkyl, das eine Nitro- oder Aminogruppe oder bis zu drei der bei der Heteroarylgruppe genannten Reste tragen kann, bevorzugt Cyclopropyl, Cyclobutyl, Cyclopentyl und Cyclohexyl;

- Tetrahydropyranyl oder Tetrahydrothiopyranyl, das jeweils eine Nitro- oder Aminogruppe oder bis zu drei der bei der Heteroarylgruppe genannten Reste tragen kann, bevorzugt Tetrahydropyran-4-yl;
- Phenyl, das eine Nitro- oder Aminogruppe oder bis zu drei der bei der Heteroarylgruppe genannten Reste tragen kann, bevorzugt Phenyl, 3-Nitrophenyl, 4-Nitrophenyl, 3-Aminophenyl, 4-Aminophenyl, Halogenphenyl wie 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl und 4-Bromphenyl, Dihalogenphenyl wie 2,3-Dichlorphenyl, 2,4-Dichlorphenyl, 2,5-Dichlorphenyl, 2,6-Dichlorphenyl, 2-Chlor-4-fluorphenyl und 2-Chlor-6-fluorphenyl, $C_1$-$C_4$-Alkylphenyl wie 4-Methylphenyl, 4-Ethylphenyl, 4-Isopropylphenyl und 4-tert.-Butylphenyl, Halogen-($C_1$-$C_4$)-alkylphenyl wie 2-Chlor-6-methylphenyl, $C_1$-$C_4$-Halogenalkylphenyl wie 2-Trifluormethylphenyl, 3-Trifluormethylphenyl, 4-Trifluormethylphenyl, $C_1$-$C_4$-Alkoxyphenyl wie 2-Methoxyphenyl, 3-Methoxyphenyl, 4-Methoxyphenyl, Di-($C_1$-$C_4$)-alkoxyphenyl wie 2,4-Dimethoxyphenyl, 3,4-Dimethoxyphenyl, Phenoxyphenyl wie 3-Phenoxyphenyl und 4-Phenoxyphenyl;
- Biphenyl, Naphthyl oder Benzyl, das jeweils eine Nitro- oder Aminogruppe oder bis zu 3 der bei der Heteroarylgruppe genannten Reste tragen kann, bevorzugt 4-Biphenyl, 1-Naphthyl, 2-Naphthyl und Benzyl;

L - Halogen wie Fluor, Chlor, Brom und Iod, bevorzugt Chlor und Brom;
- einen Rest $R$-$SO_2$-O-, wobei R die folgende Bedeutung hat:
- verzweigtes oder unverzweigtes $C_1$-$C_6$-Alkyl, insbesondere $C_1$-$C_4$-Alkyl wie Methyl, Ethyl, n-Butyl und tert.-Butyl;
- partiell oder vollständig halogeniertes $C_1$-$C_4$-Alkyl wie Trifluormethyl, Trichlormethyl, Pentafluorethyl und 2-Chlor-1,1,2-trifluorethyl;
- Phenyl, das durch Halogen wie Fluor, Chlor und Brom, $C_1$-$C_4$-Alkyl wie Methyl, Ethyl und tert.-Butyl oder Nitro substituiert sein kann.

Besonders bevorzugte Verbindungen I und III sind den Tabellen A bzw. B zu entnehmen.

Die Herstellung der Zwischenprodukte III erfolgt auf sehr vorteilhafte Weise durch an sich bekannte Oxidation von α-Halogenalkenen der Formel Va oder substituierten Propenalen der Formel Vb (vgl. G. Dittus in Houben-Weyl-Müller, Methoden der Organischen Chemie, Georg-Thieme-Verlag, Stuttgart, 1965, Bd. VI/3, Seite 385ff.) und - im Falle der Propenale -anschließender Reduktion und Veresterung der Formylgruppe:

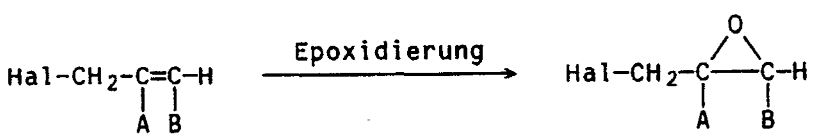


Va III (L = Hal)

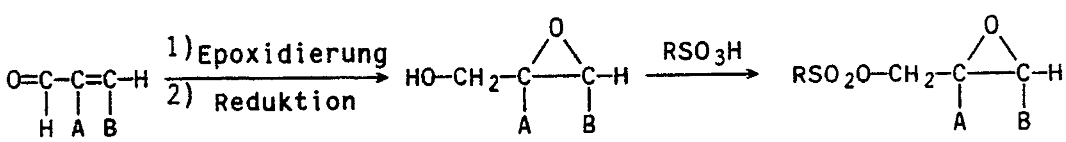


Vb III (L = $RSO_2$-O-)

Die Oxidation der Olefine Va und Vb erfolgt bevorzugt in inerten Lösungs-oder Verdünnungsmitteln wie Essigsäure, Ethylacetat, Aceton, Methyl-tert.-butylether und Dimethylformamid, insbesondere in chlorierten Kohlenwasserstoffen wie Methylenchlorid, Chloroform, Tetrachlorkohlenstoff und Dichlorethan.

Gewünschtenfalls kann in einem gepufferten Medium gearbeitet werden. Als Puffer eignen sich z.B. Natriumacetat, Natriumcarbonat, Dinatriumhydrogenphosphat und Benzyltrimethylammoniumhydroxid (Triton B).

Als Oxidationsmittel verwendet man vorteilhaft Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure und Trifluorperessigsäure.

Die Oxidation kann gewünschtenfalls katalysiert werden, z.B. mit Iod, Natriumwolframat oder Licht.

Im allgemeinen liegt die Temperatur zwischen 10 und 100 °C, insbesondere bei der Siedetemperatur des verwendeten Lösungsmittels.

Zur Oxidation eignen sich auch Alkylhydroperoxide wie tert.-Butylhydroperoxid unter Verwendung eines Katalysators wie Natriumwolframat, Perwolframsäure, Molybdänhexacarbonyl und Vanadylacetylacetonat, sowie alkalische Lösungen von z.B. 30 %igem Wasserstoffperoxid in Methanol, Ethanol, Aceton oder

Acetonitril. Bei Verwendung eines dieser genannten Oxidationsmittel, die sich z.T. in situ erzeugen lassen, empfehlen sich Temperaturen von 20 bis 30 °C.

Das Oxidationsmittel wird im allgemeinen in äquimolaren Mengen oder in einem geringen Überschuß, bis etwa 20 bis 50 %, bezogen auf die Olefine Va und Vb, eingesetzt.

Die Umsetzung wird zweckmäßig bei Normaldruck ausgeführt. Geringerer oder höherer Druck ist möglich, bringt im allgemeinen aber keine Vorteile.

Nach der Epoxidierung und anschließenden Reduktion z.B. mit Natriumborhydrid bei etwa 20 °C werden die Alkohole Vb nach an sich bekannten Verfahren in ihre Ester übergeführt (vgl. Houben-Müller-Weyl, Methoden der organischen Chemie, Georg-Thieme-Verlag Stuttgart, 1955, Bd. 9, Seiten 388, 663 und 671). Als Ester eignen sich beispielsweise Methansulfonsäureester, Trifluormethansulfonsäureester, 2,2,2-Trifluorethansulfonsäureester, Nonafluorbutansulfonsäureester, Benzolsulfonsäureester, 4-Methylbenzolsulfonsäureester, 4-Brombenzolsulfonsäureester und 4-Nitrobenzolsulfonsäureester.

Die Propenale Vb können z.B. durch an sich bekannte Kondensation von zwei Aldehyden erhalten werden.

Die Umsetzung wird zweckmäßigerweise in einem inerten Lösungs- und Verdünnungsmittel bei Temperaturen zwischen 10 und 50 °C, insbesondere 20 bis 30 °C durchgeführt.

Bezüglich des Druckes gelten die vorstehend gemachten Angaben.

Die Olefine Va sind beispielsweise durch an sich bekannte Chlorierung entsprechender Propen-Derivate $CH_3$-CA=CHB in Allylposition erhältlich.

Als Halogenierungsreagenzien sind z.B. N-Chlor- und N-Bromsuccinimid in halogenierten Kohlenwasserstoffen wie Tetrachlorkohlenstoff, Trichlorethan und Methylenchlorid geeignet. Die Temperatur liegt im allgemeinen zwischen 20 und 100 °C.

Die zu halogenierenden Propenderivate selbst sind nach an sich bekannten Methoden der Olefinsynthese erhältlich (vgl. Houben-Weyl-Müller, Methoden der organischen Chemie, Georg-Thieme Verlag Stuttgart, 1972, Bd. V/1b).

Die erfindungsgemäßen Zwischenprodukte III enthalten zwei Asymmetriezentren. Sie werden als Racemate verwendet, wie sie bei den meisten Herstellungsverfahren anfallen, können jedoch gewünschtenfalls nach den hierfür üblichen Methoden, z.B. durch Chromatographie an einem optisch aktiven Adsorptionsmittel, in die reinen Isomeren aufgetrennt werden.

Die Azolylmethyloxirane I sind auf verschiedene Weise erhältlich, und zwar vorzugsweise nach folgenden Methoden:

a) Herstellung aus einem Azol und einem Oxiran:

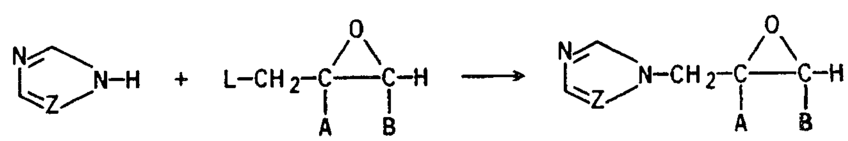

IIa III I

Bevorzugt führt man die Reaktion in einem Lösungs- oder Verdünnungsmittel durch, besonders bevorzugt unter Zusatz einer organischen oder anorganischen Base.

Als Lösungs- bzw. Verdünnungsmittel können Ketone wie Aceton, Methylethylketon und Cyclohexanon, Nitrile wie Acetonitril und Propionitril, Alkohole wie Methanol, Ethanol, Isopropanol, n-Butanol und Glykol, Ester wie Essigsäuremethylester, Essigsäureethylester und Essigsäurebutylester, Ether wie Tetrahydrofuran, Diethylether, Dimethoxyethan, Dioxan und Diisopropylether, Amide wie Dimethylformamid, Dimethylacetamid und N-Methylpyrrolidon, Dimethylsulfoxid, Sulfolan sowie Gemische der genannten Solventien verwendet werden.

Als Basen eignen sich beispielsweise Alkalimetallhydroxide wie Lithium-, Natrium- und Kaliumhydroxid, Alkalicarbonate wie Natrium-und Kaliumcarbonat, Hydrogencarbonate wie Natrium- und Kaliumhydrogencarbonat, Pyridin und 4-Dimethylaminopyridin. Es können aber auch andere übliche Basen verwendet werden.

Besonders vorteilhaft verläuft die Umsetzung in Gegenwart eines Reaktionsbeschleunigers. Für diesen Zweck eignen sich z.B. Metallhalogenide wie Natrium- und Kaliumiodid, quartäre Ammoniumsalze wie Tetrabutylammoniumchlorid, Tetrabutylammoniumbromid, Tetrabutyl ammoniumiodid, Tetrabutylammoniumhydrogensulfat, Benzyltriethylammoniumchlorid und -bromid oder Kronenether wie [12]Krone-4, [15]-Krone-5, [18]Krone-6, Dibenzo[18]krone-6 und Dicyclohexano[18]krone-6.

Zweckmäßig setzt man die Ausgangsverbindungen IIa und III im stöchiometrischen Verhältnis ein, jedoch kann in manchen Fällen auch ein Überschuß der einen oder anderen Komponente, etwa bis zu 10 %, empfehlenswert sein.

Die Umsetzung verläuft normalerweise oberhalb von 10 °C mit ausreichender Geschwindigkeit. Im allgemeinen liegt die Temperatur zwischen 10 und 150 °C, insbesondere zwischen 20 und 120 °C, bevorzugt bei der Siedetemperatur des verwendeten Lösungsmittels.

Da die Reaktion nicht druckabhängig ist, arbeitet man vorteilhaft bei Normaldruck.

b) Umsetzung eines Azolyl-Anions mit einem Oxiran

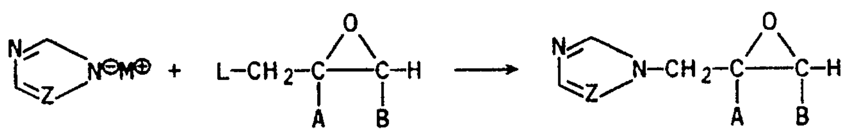

IIb III I

$M^{\oplus}$ bedeutet ein Alkalimetallkation, insbesondere Lithium, Natrium oder Kalium.

In der Regel führt man die Reaktion in einem Lösungs- oder Verdünnungsmittel durch, bevorzugt unter Zusatz einer starken anorganischen oder organischen Base.

Als Lösungs- bzw. Verdünnungsmittel können Amide wie Dimethylformamid, Diethylformamid, Dimethylacetamid und Diethylacetamid, N-Methylpyrrolidon, Hexamethyl-phosphortriamid, Sulfoxide wie Dimethylsulfoxid und Sulfolan verwendet werden.

Als Basen eignen sich beispielsweise Alkalimetallhydride wie Lithium-, Natrium- und Kaliumhydrid, Alkaliamide wie Natrium- und Kaliumamid und Alkalialkoholate wie Natrium- und Kalium-tert.-butoxid.

Die Umsetzung verläuft in der Regel oberhalb (-10) °C mit ausreichender Geschwindigkeit. Im allgemeinen liegt die Temperatur zwischen (-10) und 120 °C, insbesondere zwischen 0 und 100 °C, besonders bevorzugt zwischen 20 und 80 °C.

Bezüglich der stöchiometrischen Verhältnisse und des Druckes gelten die Angaben für Methode a).

c) Epoxidierung eines 3-Azolylprop-1-ens

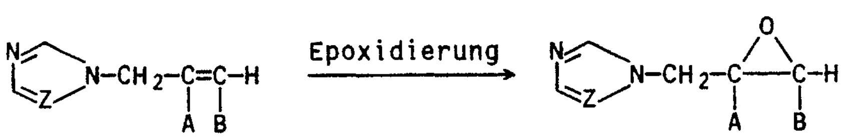


IV I

Die Epoxidierung der Olefine IV zu den Azolylmethyloxiranen I erfolgt nach an sich bekannten Methoden (vgl. G. Dittus in Houben-Weyl-Müller, Methoden der Organischen Chemie, Georg-Thieme-Verlag Stuttgart, 1965, Bd. VI/3, Seite 385ff).

Die Umsetzung erfolgt zweckmäßig in einem Lösungs- oder Verdünnungsmittel, beispielsweise in Nitrilen wie Acetonitril, Sulfoxiden wie Dimethylsulfoxid, Formamiden wie Dimethylformamid, Ketonen wie Aceton, Ethern wie Diethylether und Tetrahydrofuran sowie in Gemischen der genannten Solventien. Besonders bevorzugt sind Chlorkohlenwasserstoffe wie Methylenchlorid und Chloroform.

Im allgemeinen liegt die Reaktionstemperatur zwischen 0 und 100 °C, bevorzugt zwischen 20 und 80 °C. Besonders bevorzugt arbeitet man bei der Siedetemperatur des verwendeten Lösungsmittels.

Bezüglich der Oixidationsmittel und des Druckes gelten die Angaben für die Herstellung der Zwischenprodukte II.

Die erfindungsgemäßen Verbindungen I enthalten zwei Asymmetriezentren. Bei den meisten Herstellungsverfahren fallen sie als Racemate an, die gewünschtenfalls nach den hierfür üblichen Methoden, z.B.

durch Chromatographie an einem optisch aktiven Adsorptionsmittel, in die reinen Isomeren aufgetrennt werden können. Sowohl die Racemate als auch die reinen Isomeren sind fungizid wirksam und werden von der Erfindung umfaßt.

Als Säureadditionssalze eignen sich die Salze von solchen Säuren, welche die fungizide Wirkung von I nicht beeinträchtigen, also z.B. die Hydrochloride und -bromide, Sulfate, Nitrate, Phosphate, Oxalate oder die Dodecylbenzolsulfonate.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

Herstellungsbeispiele

Beispiel 1

cis-2-(1,2,4-Triazol-1-yl-methyl)-2-phenyl-3-(3-pyridyl)-oxiran

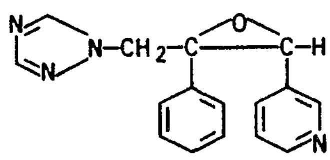

(Verbindung Nr. B.001 in Tabelle B)

Eine Mischung aus 7,8 g (0,11 mol) 1,2,4-Triazol, 150 ml N,N-Dimethylformamid und 26,3 g (0,19 mol) Kaliumcarbonat wurde 30 Minuten auf 50° C erhitzt, anschließend auf etwa 20° C gekühlt und tropfenweise mit einer Lösung von 28,2 g (0,09 mol) cis-2-(Methylsulfonyloxymethyl-2-phenyl-3-(3-pyridyl)-oxiran in 50 ml N,N-Dimethylformamid versetzt. Nach 12 Stunden Rühren bei Raumtemperatur wurde mit 100 ml Wasser versetzt, das Gemisch mehrmals mit Methyl-tert.-butylether extrahiert und die organische Phase auf das Produkt hin aufgearbeitet. Ausbeute 65 %. Fp.: 75° C.

Beispiel 2

cis-2-(1,2,4-Triazol-1-yl-methyl)-2-(4-fluorphenyl)-3-(2-methylphenyl)-oxin

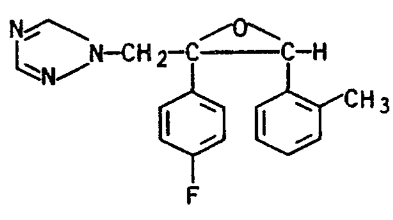

(Verbindung Nr. B.249 in Tabelle B)

Analog Beispiel 1 wurden 4,9 g (0,07 mol) 1,2,4-Triazol (in 100 ml N,N-Dimethylformamid), bei Anwesenheit von 16,4 g (0,12 mol) Kaliumcarbonat, mit 20 g (0,06 mol) cis-2-Methylsulfonyloxymethyl-2-(4-fluorphenyl)-3-(2-methylphenyl)-oxiran (in 50 ml N,N-Dimethylformamid) umgesetzt. Das Rohprodukt wurde aus Methyl-tert.-butylether/n-Hexan umkristallisiert. Ausbeute 82 %. Fp.: 99-102° C.

Vorstufe α)

2-Phenyl-3-(3-pyridyl)-propenal

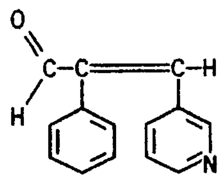

Zu einer Mischung aus 54 g (0,50 mol) Pyridin-3-carbaldehyd in 300 ml Wasser und 8,4 g (0,21 mol) Natriumhydroxid in 40 ml Wasser wurden bei 10 °C schnell 60 g (0,50 mol) Phenylacetaldehyd in der Weise zugetropft, daß die Temperatur der Lösung unter 30 °C blieb. Nach 2-stündigem Rühren bei etwa 20 °C wurden 300 ml Wasser zugegeben, die entstandene Emulsion mit Methyl-tert.-butylether ausgeschüttelt und die organische Phase auf das Produkt hin aufgearbeitet. Die Reinigung der Rohsubstanz erfolgte destillativ bei 160 °C und 0,6 mbar. Öl; Ausbeute 70,5 %.

Nach dieser Vorschrift wurde u.a. auch E-2-(4-Fluorphenyl)-3-(2-methylphenyl)-propenal durch Umsetzung von 69 g (0,50 mol) 4-Fluorphenylacetaldehyd mit 60 g (0,50 mol) 2-Methylbenzaldehyd in 300 ml Methanol hergestellt, wobei die Reinigung der Rohsubstanz destillativ bei 125 °C und 0,1 mbar erfolgte. Ausbeute 31 %.

Vorstufe β)

cis-2-Hydroxymethyl-2-phenyl-3-(3-pyridyl)-oxiran

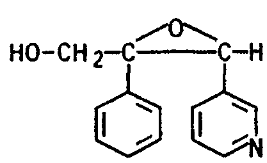

Zu einer Mischung aus 32,2 g (0,152 mol) 2-Phenyl-3-(3-pyridyl)-propenal in 150 ml Methanol und 1,7 ml konz. Natronlauge wurden bei 0 °C langsam 13,5 g 30 %ige Wasserstoffperoxid-Lösung in der Weise zugetropft, daß die Temperatur des Gemisches unter 30 °C blieb. Nach 6-stündigem Rühren bei 20 °C setzte man 6 g (0,158 mol) Natriumborhydrid, gelöst in wenig 10 %iger Natronlauge, zu und rührte die Mischung weitere 18 Stunden. Anschließend wurden 100 ml Wasser zugesetzt, die entstandene Emulsion mit Methylenchlorid ausgeschüttelt und die organische Phase auf das Produkt hin aufgearbeitet. Fp.: 108-110 °C; Ausbeute 82 %.

Nach dieser Vorschrift wurde u.a. auch cis-2-Hydroxymethyl-2-(4-fluorphenyl)-3-(2-methylphenyl)-oxiran durch Umsetzung von 37 g (0,154 mol) E-2-(4-Fluorphenyl)-3-(2-methylphenyl)-propenal mit 13,5 g Wasserstoffperoxid und anschließender Reduktion der Aldehydgruppe mit 5 g (0,132 mol) Natriumborhydrid hergestellt. Ausbeute 61 %.

Vorstufe γ) (= Zwischenprodukt der Formel III):

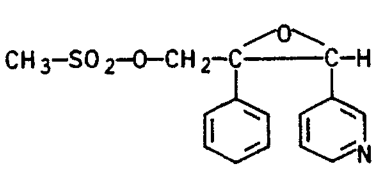

(Verbindung Nr. A.001 in Tabelle A)

17,2 g (0,15 mol) Methansulfonsäurechlorid wurden bei etwa 20 °C in eine Lösung von 28,6 g (0,12 mol) cis-2-Hydroxymethyl-2-phenyl-3-(3-pyridyl)-oxiran in 120 ml Methylenchlorid und 26 g Triethylamin eingerührt. Nachdem diese Mischung 24 Stunden gerührt worden war, wurde sie mit wäßriger Natriumhydrogencarbonat-Lösung und Wasser gewaschen und auf das Produkt hin aufgearbeitet. Ausbeute: 69 %.

Nach dieser Vorschrift wurde u.a. cis-2-(Methylsulfonyloxymethyl)-2-(4-fluorphenyl)-3-(2-methylphenyl)-oxiran (Verbindung Nr. A.117 in Tabelle A) durch Umsetzung von 13,4 g (0,117 mol) Methansulfonsäurechlorid mit 24 g (0,093 mol) cis-2-Hydroxymethyl-2-(4-fluorphenyl)-3-(2-methylphenyl)-oxiran in 100 ml Methylenchlorid hergestellt. Ausbeute: 75 %.

In Tabelle A sind weitere Zwischenprodukte III, in Tabelle B weitere Azolylmethyloxirane I enthalten, die sich analog den Beispielsverbindungen herstellen lassen.

Tabelle A

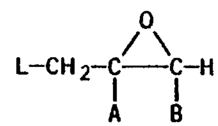

(III)

| Nr. | A | B | L | Fp. [°C]/$^1$H-NMR [ppm] |
|---|---|---|---|---|
| A.001 | $C_6H_5$ | 3-Pyridyl | $OSO_2CH_3$ | 108-110 |
| A.002 | $C_6H_5$ | 2-Thienyl | $OSO_2CH_3$ | |
| A.003 | $C_6H_5$ | 2-Thienyl | Cl | |
| A.004 | $C_6H_5$ | 2-Thienyl | Br | |
| A.005 | $C_6H_5$ | 3-Thienyl | $OSO_2CH_3$ | |
| A.006 | $C_6H_5$ | 2-Furyl | $OSO_2CH_3$ | |
| A.007 | $C_6H_5$ | 2-Pyrryl | $OSO_2CH_3$ | |
| A.008 | $C_6H_5$ | 4-Oxazolyl | $OSO_2CH_3$ | |
| A.009 | $C_6H_5$ | 4-Thiazolyl | $OSO_2CH_3$ | |
| A.010 | $C_6H_5$ | 5-Oxazolyl | $OSO_2CH_3$ | |
| A.011 | $C_6H_5$ | 3-Isoxazolyl | $OSO_2CH_3$ | |
| A.012 | $C_6H_5$ | 4-Isoxazolyl | $OSO_2CH_3$ | |
| A.013 | $C_6H_5$ | 5-Isoxazolyl | $OSO_2CH_3$ | |
| A.014 | $C_6H_5$ | 4-Imidazolyl | $OSO_2CH_3$ | |
| A.015 | $C_6H_5$ | 5-Thiazolyl | $OSO_2CH_3$ | |
| A.016 | 2-Cl-$C_6H_4$ | 2-Thienyl | $OSO_2CH_3$ | |
| A.017 | 4-Cl-$C_6H_4$ | 3-Pyridyl | $OSO_2CH_3$ | |
| A.018 | 4-Cl-$C_6H_4$ | 2-Thienyl | $OSO_2CH_3$ | |
| A.019 | 4-Cl-$C_6H_4$ | 3-Thienyl | $OSO_2CH_3$ | |
| A.020 | 4-Cl-$C_6H_4$ | 2-Furyl | $OSO_2CH_3$ | |

Tabelle A (Fortsetzung)

| Nr. | A | B | L | Fp. [°C]/$^1$H-NMR [ppm] |
|---|---|---|---|---|
| A.021 | 4-Cl-$C_6H_4$ | 4-Pyridyl | $OSO_2CH_3$ | |
| A.022 | 2,4-$Cl_2$-$C_6H_3$ | 2-Thienyl | $OSO_2CH_3$ | |
| A.023 | 2,4-$Cl_2$-$C_6H_3$ | 2-Furyl | $OSO_2CH_3$ | |
| A.024 | 2-F-$C_6H_4$ | 2-Thienyl | $OSO_2CH_3$ | |
| A.025 | 2-F-$C_6H_4$ | 3-Pyridyl | $OSO_2CH_3$ | |
| A.026 | 4-F-$C_6H_4$ | 2-Thienyl | $OSO_2CH_3$ | |
| A.027 | 4-F-$C_6H_4$ | 3-Thienyl | $OSO_2CH_3$ | 4,68, 4,39(2d); 4,10(s); 2,84(s) |
| A.028 | 4-F-$C_6H_4$ | 2-Furyl | $OSO_2CH_3$ | |
| A.029 | 4-F-$C_6H_4$ | 2-Pyridyl | $OSO_2CH_3$ | |
| A.030 | 4-F-$C_6H_4$ | 3-Pyridyl | $OSO_2CH_3$ | |
| A.031 | 4-F-$C_6H_4$ | 4-Pyridyl | $OSO_2CH_3$ | |
| A.032 | 4-F-$C_6H_4$ | 2-Pyrrolyl | $OSO_2CH_3$ | |
| A.033 | 4-F-$C_6H_4$ | 4-Oxazolyl | $OSO_2CH_3$ | |
| A.034 | 4-F-$C_6H_4$ | 4-Thiazolyl | $OSO_2CH_3$ | |
| A.035 | 4-F-$C_6H_4$ | 5-Oxazolyl | $OSO_2CH_3$ | |
| A.036 | 4-F-$C_6H_4$ | 3-Isoxazolyl | $OSO_2CH_3$ | |
| A.037 | 4-F-$C_6H_4$ | 4-Isoxazolyl | $OSO_2CH_3$ | |
| A.038 | 4-F-$C_6H_4$ | 5-Isoxazolyl | $OSO_2CH_3$ | |
| A.039 | 4-F-$C_6H_4$ | 4-Imidazolyl | $OSO_2CH_3$ | |
| A.040 | 4-Br-$C_6H_4$ | 2-Thienyl | $OSO_2CH_3$ | |
| A.041 | 4-$CF_3$-$C_6H_4$ | 3-Thienyl | $OSO_2CH_3$ | |
| A.042 | 4-$CH_3$-$C_6H_4$ | 2-Furyl | $OSO_2CH_3$ | |
| A.043 | 4-$OCH_3$-$C_6H_4$ | 2-Thienyl | $OSO_2CH_3$ | |
| A.044 | 4-Biphenyl | 3-Pyridyl | $OSO_2CH_3$ | |

Tabelle A (Fortsetzung)

| Nr. | A | B | L | Fp. [°C]/$^1$H-NMR [ppm] |
|---|---|---|---|---|
| A.045 | 2-Thienyl | $C_6H_5$ | $OSO_2CH_3$ | |
| A.046 | 2-Thienyl | $2-Cl-C_6H_4$ | $OSO_2CH_3$ | |
| A.047 | 2-Thienyl | $4-Cl-C_6H_4$ | $OSO_2CH_3$ | |
| A.048 | 2-Thienyl | $2,4-Cl_2-C_6H_3$ | $OSO_2CH_3$ | |
| A.049 | 2-Thienyl | $4-F-C_6H_4$ | $OSO_2CH_3$ | |
| A.050 | 2-Thienyl | $2-CF_3-C_6H_4$ | $OSO_2CH_3$ | |
| A.051 | 2-Thienyl | $2-CH_3-C_6H_4$ | $OSO_2CH_3$ | |
| A.052 | 2-Thienyl | 2-Thienyl | $OSO_2CH_3$ | |
| A.053 | 2-Thienyl | 3-Thienyl | $OSO_2CH_3$ | |
| A.054 | 2-Thienyl | 3-Pyridyl | $OSO_2CH_3$ | |
| A.055 | 2-Thienyl | 2-Furyl | $OSO_2CH_3$ | |
| A.056 | 3-Thienyl | $C_6H_5$ | $OSO_2CH_3$ | |
| A.057 | 3-Thienyl | $2-Cl-C_6H_4$ | $OSO_2CH_3$ | |
| A.058 | 3-Thienyl | $4-Cl-C_6H_4$ | $OSO_2CH_3$ | |
| A.059 | 3-Thienyl | $2,4-Cl_2-C_6H_3$ | $OSO_2CH_3$ | |
| A.060 | 3-Thienyl | $4-F-C_6H_4$ | $OSO_2CH_3$ | |
| A.061 | 3-Thienyl | $2-CF_3-C_6H_4$ | $OSO_2CH_3$ | |
| A.062 | 3-Thienyl | $2-CH_3-C_6H_4$ | $OSO_2CH_3$ | |
| A.063 | 3-Thienyl | 2-Thienyl | $OSO_2CH_3$ | |
| A.064 | 3-Thienyl | 3-Thienyl | $OSO_2CH_3$ | |
| A.065 | 3-Thienyl | 3-Pyridyl | $OSO_2CH_3$ | |
| A.066 | 3-Thienyl | 2-Furyl | $OSO_2CH_3$ | |
| A.067 | 2-Furyl | $C_6H_5$ | $OSO_2CH_3$ | |
| A.068 | 2-Furyl | $2-Cl-C_6H_4$ | $OSO_2CH_3$ | |
| A.069 | 2-Furyl | $4-Cl-C_6H_4$ | $OSO_2CH_3$ | |

Tabelle A (Fortsetzung)

| Nr. | A | B | L | Fp. [°C]/$^1$H-NMR [ppm] |
|---|---|---|---|---|
| A.070 | 2-Furyl | 4-F-$C_6H_4$ | $OSO_2CH_3$ | |
| A.071 | 2-Furyl | 3-Pyridyl | $OSO_2CH_3$ | |
| A.072 | 2-Furyl | 4-Oxazolyl | $OSO_2CH_3$ | |
| A.073 | 3-Pyridyl | $C_6H_5$ | $OSO_2CH_3$ | |
| A.074 | 3-Pyridyl | 2-Cl-$C_6H_4$ | $OSO_2CH_3$ | |
| A.075 | 3-Pyridyl | 4-F-$C_6H_4$ | $OSO_2CH_3$ | |
| A.076 | 3-Pyridyl | 3-Pyridyl | $OSO_2CH_3$ | |
| A.077 | 4-Pyridyl | $C_6H_5$ | $OSO_2CH_3$ | |
| A.078 | 4-Pyridyl | 2-Cl-$C_6H_4$ | $OSO_2CH_3$ | |
| A.079 | 4-Pyridyl | 4-Cl-$C_6H_4$ | $OSO_2CH_3$ | |
| A.080 | 4-Pyridyl | 2-$CH_3$-$C_6H_4$ | $OSO_2CH_3$ | |
| A.081 | 4-Pyridyl | 2-$CF_3$-$C_6H_4$ | $OSO_2CH_3$ | |
| A.082 | 4-Pyridyl | 2-Thienyl | $OSO_2CH_3$ | |
| A.083 | 4-Pyridyl | 3-Thienyl | $OSO_2CH_3$ | |
| A.084 | 4-Pyridyl | 2-Furyl | $OSO_2CH_3$ | |
| A.085 | 2-Pyridyl | $C_6H_5$ | $OSO_2CH_3$ | |
| A.086 | 2-Pyridyl | 2-Cl-$C_6H_4$ | $OSO_2CH_3$ | |
| A.087 | 2-Pyridyl | 2-$CH_3$-$C_6H_4$ | $OSO_2CH_3$ | |
| A.088 | 2-Pyridyl | 2-$CF_3$-$C_6H_4$ | $OSO_2CH_3$ | |
| A.089 | 2-Pyridyl | 2-Thienyl | $OSO_2CH_3$ | |
| A.090 | 2-Pyridyl | 3-Thienyl | $OSO_2CH_3$ | |
| A.091 | 4-Oxazolyl | $C_6H_5$ | $OSO_2CH_3$ | |
| A.092 | 4-Oxazolyl | 2-Cl-$C_6H_4$ | $OSO_2CH_3$ | |
| A.093 | 4-Thiazolyl | $C_6H_5$ | $OSO_2CH_3$ | |
| A.094 | 4-Thiazolyl | 2-Cl-$C_6H_4$ | $OSO_2CH_3$ | |

Tabelle A (Fortsetzung)

| Nr. | A | B | L | Fp. [°C]/$^1$H-NMR [ppm] |
|---|---|---|---|---|
| A.095 | 5-Oxazolyl | $C_6H_5$ | $OSO_2CH_3$ | |
| A.096 | 5-Oxazolyl | 2-Cl-$C_6H_4$ | $OSO_2CH_3$ | |
| A.097 | 5-Thiazolyl | $C_6H_5$ | $OSO_2CH_3$ | |
| A.098 | 5-Thiazolyl | 2-Cl-$C_6H_4$ | $OSO_2CH_3$ | |
| A.099 | 3-Isoxazolyl | $C_6H_5$ | $OSO_2CH_3$ | |
| A.100 | 3-Isoxazolyl | 2-Cl-$C_6H_4$ | $OSO_2CH_3$ | |
| A.101 | 4-Isoxazolyl | $C_6H_5$ | $OSO_2CH_3$ | |
| A.102 | 4-Isoxazolyl | 2-Cl-$C_6H_4$ | $OSO_2CH_3$ | |
| A.103 | 5-Isoxazolyl | $C_6H_5$ | $OSO_2CH_3$ | |
| A.104 | 5-Isoxazolyl | 2-Cl-$C_6H_4$ | $OSO_2CH_3$ | |
| A.105 | 4-Imidazolyl | $C_6H_5$ | $OSO_2CH_3$ | |
| A.106 | 4-Imidazolyl | 2-Cl-$C_6H_4$ | $OSO_2CH_3$ | |
| A.107 | Cyclohexyl | 2-Thienyl | $OSO_2CH_3$ | |
| A.108 | Cyclohexyl | 3-Thienyl | $OSO_2CH_3$ | |
| A.109 | Cyclohexyl | 2-Furyl | $OSO_2CH_3$ | |
| A.110 | Cyclohexyl | 3-Pyridyl | $OSO_2CH_3$ | |
| A.111 | tert.-$C_4H_9$ | 2-Thienyl | $OSO_2CH_3$ | |
| A.112 | tert.-$C_4H_9$ | 3-Thienyl | $OSO_2CH_3$ | |
| A.113 | tert.-$C_4H_9$ | 2-Furyl | $OSO_2CH_3$ | |
| A.114 | tert.-$C_4H_9$ | 3-Pyridyl | $OSO_2CH_3$ | |
| A.115 | 4-F-$C_6H_4$ | 2-$CH_3$-$C_6H_4$ | Cl | |
| A.116 | 4-F-$C_6H_4$ | 2-$CH_3$-$C_6H_4$ | Br | |
| A.117 | 4-F-$C_6H_4$ | 2-$CH_3$-$C_6H_4$ | $OSO_2CH_3$ | 2,20(s), 2,62(s), 4,04(s), 4,18, 4,42(2d) |
| A.118 | 3-F-$C_6H_4$ | 2-$CH_3$-$C_6H_4$ | Cl | |

Tabelle A (Fortsetzung)

| Nr. | A | B | L | Fp. [°C]/$^1$H-NMR [ppm] |
|---|---|---|---|---|
| A.119 | $3-F-C_6H_4$ | $2-CH_3-C_6H_4$ | Br | |
| A.120 | $3-F-C_6H_4$ | $2-CH_3-C_6H_4$ | $OSO_2CH_3$ | |
| A.121 | $2-F-C_6H_4$ | $2-CH_3-C_6H_4$ | Cl | |
| A.122 | $2-F-C_6H_4$ | $2-CH_3-C_6H_4$ | Br | |
| A.123 | $2-F-C_6H_4$ | $2-CH_3-C_6H_4$ | $OSO_2CH_3$ | |
| A.124 | $2-Cl-C_6H_4$ | $2-CH_3-C_6H_4$ | Cl | |
| A.125 | $2-Cl-C_6H_4$ | $2-CH_3-C_6H_4$ | Br | |
| A.126 | $2-Cl-C_6H_4$ | $2-CH_3-C_6H_4$ | $OSO_2CH_3$ | |
| A.127 | $3-Cl-C_6H_4$ | $2-CH_3-C_6H_4$ | Cl | |
| A.128 | $3-Cl-C_6H_4$ | $2-CH_3-C_6H_4$ | Br | |
| A.129 | $3-Cl-C_6H_4$ | $2-CH_3-C_6H_4$ | $OSO_2CH_3$ | |
| A.130 | $4-Cl-C_6H_4$ | $2-CH_3-C_6H_4$ | Cl | |
| A.131 | $4-Cl-C_6H_4$ | $2-CH_3-C_6H_4$ | Br | |
| A.132 | $4-Cl-C_6H_4$ | $2-CH_3-C_6H_4$ | $OSO_2CH_3$ | 2,30(s),2,64(s),4,06(s),4,18, 4,44(2d) |
| A.133 | $2,4-Cl_2-C_6H_3$ | $2-CH_3-C_6H_4$ | Cl | |
| A.134 | $2,4-Cl_2-C_6H_3$ | $2-CH_3-C_6H_4$ | Br | |
| A.135 | $2,4-Cl_2-C_6H_3$ | $2-CH_3-C_6H_4$ | $OSO_2CH_3$ | |
| A.136 | $4-Br-C_6H_4$ | $2-CH_3-C_6H_4$ | Cl | |
| A.137 | $4-Br-C_6H_4$ | $2-CH_3-C_6H_4$ | Br | |
| A.138 | $4-Br-C_6H_4$ | $2-CH_3-C_6H_4$ | $OSO_2CH_3$ | |
| A.139 | $2-CH_3-C_6H_4$ | $2-CH_3-C_6H_4$ | Cl | |
| A.140 | $2-CH_3-C_6H_4$ | $2-CH_3-C_6H_4$ | Br | |
| A.141 | $2-CH_3-C_6H_4$ | $2-CH_3-C_6H_4$ | $OSO_2CH_3$ | |
| A.142 | $4-CH_3-C_6H_4$ | $2-CH_3-C_6H_4$ | Cl | |

Tabelle A (Fortsetzung)

| Nr. | A | B | L | Fp. [°C]/$^{1}$H-NMR [ppm] |
|---|---|---|---|---|
| A.143 | 4-$CH_3$-$C_6H_4$ | 2-$CH_3$-$C_6H_4$ | Br | |
| A.144 | 4-$CH_3$-$C_6H_4$ | 2-$CH_3$-$C_6H_4$ | $OSO_2CH_3$ | |
| A.145 | 2-$CF_3$-$C_6H_4$ | 2-$CH_3$-$C_6H_4$ | Cl | |
| A.146 | 2-$CF_3$-$C_6H_4$ | 2-$CH_3$-$C_6H_4$ | Br | |
| A.147 | 2-$CF_3$-$C_6H_4$ | 2-$CH_3$-$C_6H_4$ | $OSO_2CH_3$ | |
| A.148 | 3-$CF_3$-$C_6H_4$ | 2-$CH_3$-$C_6H_4$ | Cl | |
| A.149 | 3-$CF_3$-$C_6H_4$ | 2-$CH_3$-$C_6H_4$ | Br | |
| A.150 | 3-$CF_3$-$C_6H_4$ | 2-$CH_3$-$C_6H_4$ | $OSO_2CH_3$ | |
| A.151 | 4-$CF_3$-$C_6H_4$ | 2-$CH_3$-$C_6H_4$ | Cl | |
| A.152 | 4-$CF_3$-$C_6H_4$ | 2-$CH_3$-$C_6H_4$ | Br | |
| A.153 | 4-$CF_3$-$C_6H_4$ | 2-$CH_3$-$C_6H_4$ | $OSO_2CH_3$ | |
| A.154 | 4-$OCH_3$-$C_6H_4$ | 2-$CH_3$-$C_6H_4$ | Cl | |
| A.155 | 4-$OCH_3$-$C_6H_4$ | 2-$CH_3$-$C_6H_4$ | Br | |
| A.156 | 4-$OCH_3$-$C_6H_4$ | 2-$CH_3$-$C_6H_4$ | $OSO_2CH_3$ | |
| A.157 | $C_6H_5$ | 2-$CH_3$-$C_6H_4$ | Cl | |
| A.158 | $C_6H_5$ | 2-$CH_3$-$C_6H_4$ | Br | |
| A.159 | $C_6H_5$ | 2-$CH_3$-$C_6H_4$ | $OSO_2CH_3$ | 2,30(s),2,62(s),4,04(s),4,16, 4,42(2d) |
| A.160 | 1-Naphthyl | 2-$CH_3$-$C_6H_4$ | Cl | |
| A.161 | 1-Naphthyl | 2-$CH_3$-$C_6H_4$ | Br | |
| A.162 | 1-Naphthyl | 2-$CH_3$-$C_6H_4$ | $OSO_2CH_3$ | |
| A.163 | $CH_3$ | 2-$CH_3$-$C_6H_4$ | Cl | |
| A.164 | $CH_3$ | 2-$CH_3$-$C_6H_4$ | Br | |
| A.165 | $CH_3$ | 2-$CH_3$-$C_6H_4$ | $OSO_2CH_3$ | 1,64(s),2,24(s),2,68(s),3,82, 3,94(2d),4,20(s) |

Tabelle A (Fortsetzung)

| Nr. | A | B | L | Fp. [°C]/$^1$H-NMR [ppm] |
|---|---|---|---|---|
| A.166 | tert.-$C_4H_9$ | 2-$CH_3$-$C_6H_4$ | Cl | |
| A.167 | tert.-$C_4H_9$ | 2-$CH_3$-$C_6H_4$ | Br | |
| A.168 | tert.-$C_4H_9$ | 2-$CH_3$-$C_6H_4$ | $OSO_2CH_3$ | |
| A.169 | Cyclohexyl | 2-$CH_3$-$C_6H_4$ | Cl | |
| A.170 | Cyclohexyl | 2-$CH_3$-$C_6H_4$ | Br | |
| A.171 | Cyclohexyl | 2-$CH_3$-$C_6H_4$ | $OSO_2CH_3$ | |
| A.172 | 4-Tetrahydropyranyl | 2-$CH_3$-$C_6H_4$ | Cl | |
| A.173 | 4-Tetrahydropyranyl | 2-$CH_3$-$C_6H_4$ | Br | |
| A.174 | 4-Tetrahydropyranyl | 2-$CH_3$-$C_6H_4$ | $OSO_2CH_3$ | |

Tabelle B

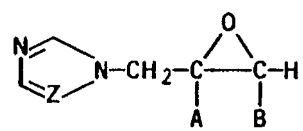

(I)

| Nr. | A | B | Z | Fp. [°C]/IR [$cm^{-1}$] |
|---|---|---|---|---|
| B.001 | Phenyl | 3-Pyridyl | N | 75 |
| B.002 | Phenyl | 2-Pyridyl | N | |
| B.003 | Phenyl | 4-Pyridyl | N | |
| B.004 | Phenyl | 2-Thienyl | N | |
| B.005 | Phenyl | 2-Thienyl | CH | |
| B.006 | Phenyl | 3-Thienyl | N | |
| B.007 | Phenyl | 3-Thienyl | CH | |
| B.008 | Phenyl | 2-Furyl | N | |
| B.009 | Phenyl | 2-Furyl | CH | |
| B.010 | Phenyl | 2-Pyrryl | N | |
| B.011 | Phenyl | 3-Pyrryl | N | |
| B.012 | Phenyl | 4-Oxazolyl | N | |
| B.013 | Phenyl | 4-Thiazolyl | N | |
| B.014 | Phenyl | 5-Oxazolyl | N | |
| B.015 | Phenyl | 3-Isoxazolyl | N | |
| B.016 | Phenyl | 4-Isoxazolyl | N | |
| B.017 | Phenyl | 5-Isoxazolyl | N | |
| B.018 | Phenyl | 4-Imidazolyl | N | |
| B.019 | Phenyl | 4-Imidazolyl | CH | |
| B.020 | Phenyl | 5-Thiazolyl | N | |

Tabelle B (Fortsetzung)

| Nr. | A | B | Z | Fp. [°C]/IR [$cm^{-1}$] |
|---|---|---|---|---|
| B.021 | $2-Cl-C_6H_4$ | 2-Pyridyl | N | |
| B.022 | $2-Cl-C_6H_4$ | 2-Thienyl | N | |
| B.023 | $2-Cl-C_6H_4$ | 3-Thienyl | N | |
| B.024 | $2-Cl-C_6H_4$ | 2-Furyl | N | |
| B.025 | $2-Cl-C_6H_4$ | 2-Pyrryl | N | |
| B.026 | $2-Cl-C_6H_4$ | 4-Thiazolyl | N | |
| B.027 | $2-Cl-C_6H_4$ | 4-Isoxazolyl | N | |
| B.028 | $4-Cl-C_6H_4$ | 2-Pyridyl | N | |
| B.029 | $4-Cl-C_6H_4$ | 3-Pyridyl | N | |
| B.030 | $4-Cl-C_6H_4$ | 4-Pyridyl | N | |
| B.031 | $4-Cl-C_6H_4$ | 2-Thienyl | N | |
| B.032 | $4-Cl-C_6H_4$ | 3-Thienyl | N | |
| B.033 | $4-Cl-C_6H_4$ | 2-Furyl | N | |
| B.034 | $4-Cl-C_6H_4$ | 2-Pyrrolyl | N | |
| B.035 | $4-Cl-C_6H_4$ | 4-Oxazolyl | N | |
| B.036 | $4-Cl-C_6H_4$ | 5-Oxazolyl | N | |
| B.037 | $4-Cl-C_6H_4$ | 4-Thiazolyl | N | |
| B.038 | $4-Cl-C_6H_4$ | 4-Imidazolyl | N | |
| B.039 | $2,4-Cl_2-C_6H_3$ | 2-Pyridyl | N | |
| B.040 | $2,4-Cl_2-C_6H_3$ | 3-Pyridyl | N | |
| B.041 | $2,4-Cl_2-C_6H_3$ | 2-Thienyl | N | |
| B.042 | $2,4-Cl_2-C_6H_3$ | 3-Thienyl | N | |
| B.043 | $2,4-Cl_2-C_6H_3$ | 2-Furyl | N | |
| B.044 | $2,4-Cl_2-C_6H_3$ | 2-Pyrrolyl | N | |

Tabelle B (Fortsetzung)

| Nr. | A | B | Z | Fp. [°C]/IR [$cm^{-1}$] |
|---|---|---|---|---|
| B.045 | 2,4-$Cl_2$-$C_6H_3$ | 4-Thiazolyl | N | |
| B.046 | 2,4-$Cl_2$-$C_6H_3$ | 4-Isoxazolyl | N | |
| B.047 | 2-F-$C_6H_4$ | 3-Pyridyl | N | |
| B.048 | 2-F-$C_6H_4$ | 2-Thienyl | N | |
| B.049 | 2-F-$C_6H_4$ | 3-Thienyl | N | |
| B.050 | 2-F-$C_6H_4$ | 2-Furyl | N | |
| B.051 | 2-F-$C_6H_4$ | 2-Pyrrolyl | N | |
| B.052 | 4-F-$C_6H_4$ | 2-Pyridyl | N | |
| B.053 | 4-F-$C_6H_4$ | 3-Pyridyl | N | |
| B.054 | 4-F-$C_6H_4$ | 4-Pyridyl | N | |
| B.055 | 4-F-$C_6H_4$ | 2-Thienyl | N | |
| B.056 | 4-F-$C_6H_4$ | 2-Thienyl | CH | |
| B.057 | 4-F-$C_6H_4$ | 3-Thienyl | N | 78- 80 |
| B.058 | 4-F-$C_6H_4$ | 3-Thienyl | CH | 1607, 1513, 1226, 841 |
| B.059 | 4-F-$C_6H_4$ | 2-Furyl | N | |
| B.060 | 4-F-$C_6H_4$ | 2-Furyl | CH | |
| B.061 | 4-F-$C_6H_4$ | 4-Pyrrolyl | N | |
| B.062 | 4-F-$C_6H_4$ | 3-Pyrrolyl | N | |
| B.063 | 4-F-$C_6H_4$ | 4-Oxazolyl | N | |
| B.064 | 4-F-$C_6H_4$ | 4-Oxazolyl | CH | |
| B.065 | 4-F-$C_6H_4$ | 5-Oxazolyl | N | |
| B.066 | 4-F-$C_6H_4$ | 5-Oxazolyl | CH | |
| B.067 | 4-F-$C_6H_4$ | 4-Thiazolyl | N | |
| B.068 | 4-F-$C_6H_4$ | 4-Thiazolyl | CH | |

Tabelle B (Fortsetzung)

| Nr. | A | B | Z | Fp. [°C]/IR [$cm^{-1}$] |
|---|---|---|---|---|
| B.069 | 4-F-$C_6H_4$ | 5-Thiazolyl | N | |
| B.070 | 4-F-$C_6H_4$ | 5-Thiazolyl | CH | |
| B.071 | 4-F-$C_6H_4$ | 3-Isoxazolyl | N | |
| B.072 | 4-F-$C_6H_4$ | 4-Isoxazolyl | N | |
| B.073 | 4-F-$C_6H_4$ | 5-Isoxazolyl | N | |
| B.074 | 4-F-$C_6H_4$ | 4-Imidazolyl | N | |
| B.075 | 4-Br-$C_6H_4$ | 3-Pyridyl | N | |
| B.076 | 4-Br-$C_6H_4$ | 2-Thienyl | N | |
| B.077 | 4-Br-$C_6H_4$ | 3-Thienyl | N | |
| B.078 | 4-Br-$C_6H_4$ | 2-Furyl | N | |
| B.079 | 4-Br-$C_6H_4$ | 4-Isoxazolyl | N | |
| B.080 | 4-$CF_3$-$C_6H_4$ | 3-Pyridyl | N | |
| B.081 | 4-$CF_3$-$C_6H_4$ | 2-Thienyl | N | |
| B.082 | 4-$CF_3$-$C_6H_4$ | 3-Thienyl | N | |
| B.083 | 4-$CF_3$-$C_6H_4$ | 2-Furyl | N | |
| B.084 | 4-$CF_3$-$C_6H_4$ | 4-Isoxazolyl | N | |
| B.085 | 4-$CH_3$-$C_6H_4$ | 3-Pyridyl | N | |
| B.086 | 4-$CH_3$-$C_6H_4$ | 2-Thienyl | N | |
| B.087 | 4-$CH_3$-$C_6H_4$ | 3-Thienyl | N | |
| B.088 | 4-$CH_3$-$C_6H_4$ | 2-Furyl | N | |
| B.089 | 4-$CH_3$-$C_6H_4$ | 3-Isoxazolyl | N | |
| B.090 | 4-$CH_3$-$C_6H_4$ | 4-Thiazolyl | N | |
| B.091 | 4-$OCH_3$-$C_6H_4$ | 3-Pyridyl | N | |
| B.092 | 4-$OCH_3$-$C_6H_4$ | 2-Thienyl | N | |

Tabelle B (Fortsetzung)

| Nr. | A | B | Z | Fp. [°C]/IR [$cm^{-1}$] |
|---|---|---|---|---|
| B.093 | 4-$OCH_3$-$C_6H_4$ | 3-Thienyl | N | |
| B.094 | 4-$OCH_3$-$C_6H_4$ | 4-Oxazolyl | N | |
| B.095 | 4-$OCH_3$-$C_6H_4$ | 2-Furyl | N | |
| B.096 | 4-Biphenyl | 3-Pyridyl | N | |
| B.097 | 4-Biphenyl | 2-Thienyl | N | |
| B.098 | 2-Thienyl | $C_6H_5$ | N | |
| B.099 | 2-Thienyl | $C_6H_5$ | CH | |
| B.100 | 2-Thienyl | 2-Cl-$C_6H_4$ | N | 119-121 |
| B.101 | 2-Thienyl | 2-Cl-$C_6H_4$ | N x $CuSO_4$ | |
| B.102 | 2-Thienyl | 2-Cl-$C_6H_4$ | CH | |
| B.103 | 2-Thienyl | 4-Cl-$C_6H_4$ | N | |
| B.104 | 2-Thienyl | 4-Cl-$C_6H_4$ | CH | |
| B.105 | 2-Thienyl | 2,4-$Cl_2$-$C_6H_3$ | N | |
| B.106 | 2-Thienyl | 2,4-$Cl_2$-$C_6H_3$ | CH | |
| B.107 | 2-Thienyl | 2-Br-$C_6H_4$ | N | |
| B.108 | 2-Thienyl | 2-F-$C_6H_4$ | N | |
| B.109 | 2-Thienyl | 4-F-$C_6H_4$ | N | |
| B.110 | 2-Thienyl | 4-Br-$C_6H_4$ | N | |
| B.111 | 2-Thienyl | 2-$CH_3$-$C_6H_4$ | N | |
| B.112 | 2-Thienyl | 2-$CH_3$-$C_6H_4$ | CH | |
| B.113 | 2-Thienyl | 4-$CH_3$-$C_6H_4$ | N | |
| B.114 | 2-Thienyl | 2,4-$(CH_3)_2$-$C_6H_3$ | N | |
| B.115 | 2-Thienyl | 2-$CF_3$-$C_6H_4$ | N | |
| B.116 | 2-Thienyl | 2-$CF_3$-$C_6H_4$ | CH | |

Tabelle B (Fortsetzung)

| Nr. | A | B | Z | Fp. [°C]/IR [$cm^{-1}$] |
|---|---|---|---|---|
| B.117 | 2-Thienyl | $4-CF_3-C_6H_4$ | N | |
| B.118 | 2-Thienyl | $2-OCH_3-C_6H_4$ | N | |
| B.119 | 2-Thienyl | $4-NO_2-C_6H_4$ | N | |
| B.120 | 2-Thienyl | 2-Naphthyl | N | |
| B.121 | 2-Thienyl | 2-Thienyl | N | |
| B.122 | 2-Thienyl | 3-Thienyl | N | |
| B.123 | 2-Thienyl | 3-Pyridyl | N | |
| B.124 | 2-Thienyl | 2-Furyl | N | |
| B.125 | 2-Thienyl | 4-Isoxazolyl | N | |
| B.126 | 2-Thienyl | $CH_3$ | N | |
| B.127 | 3-Thienyl | $C_6H_5$ | N | |
| B.128 | 3-Thienyl | $C_6H_5$ | CH | |
| B.129 | 3-Thienyl | $2-Cl-C_6H_4$ | N | |
| B.130 | 3-Thienyl | $2-Cl-C_6H_4$ | CH | |
| B.131 | 3-Thienyl | $4-Cl-C_6H_4$ | N | |
| B.132 | 3-Thienyl | $2,4-Cl_2-C_6H_3$ | N | |
| B.133 | 3-Thienyl | $2-Br-C_6H_4$ | N | |
| B.134 | 3-Thienyl | $4-Br-C_6H_4$ | N | |
| B.135 | 3-Thienyl | $2-F-C_6H_4$ | N | |
| B.136 | 3-Thienyl | $4-F-C_6H_4$ | N | |
| B.137 | 3-Thienyl | $2-CH_3-C_6H_4$ | N | |
| B.138 | 3-Thienyl | $2-CH_3-C_6H_4$ | CH | |
| B.139 | 3-Thienyl | $2,4-(CH_3)_2-C_6H_3$ | N | |
| B.140 | 3-Thienyl | $2-CF_3-C_6H_4$ | N | |

Tabelle B (Fortsetzung)

| Nr. | A | B | Z | Fp. [°C]/IR [$cm^{-1}$] |
|---|---|---|---|---|
| B.141 | 3-Thienyl | $2-CF_3-C_6H_4$ | CH | |
| B.142 | 3-Thienyl | $4-CF_3-C_6H_4$ | N | |
| B.143 | 3-Thienyl | $2-OCH_3-C_6H_4$ | N | |
| B.144 | 3-Thienyl | $4-NO_2-C_6H_4$ | N | |
| B.145 | 3-Thienyl | 2-Naphthyl | N | |
| B.146 | 3-Thienyl | 2-Thienyl | N | |
| B.147 | 3-Thienyl | 3-Thienyl | N | |
| B.148 | 3-Thienyl | 3-Pyridyl | N | |
| B.149 | 3-Thienyl | 2-Furyl | N | |
| B.150 | 3-Thienyl | 4-Oxazolyl | N | |
| B.151 | 3-Thienyl | 4-Thiazolyl | N | |
| B.152 | 3-Thienyl | tert.-$C_4H_9$ | N | |
| B.153 | 2-Furyl | $C_6H_5$ | N | |
| B.154 | 2-Furyl | $C_6H_5$ | CH | |
| B.155 | 2-Furyl | $2-Cl-C_6H_4$ | N | |
| B.156 | 2-Furyl | $2-Cl-C_6H_4$ | CH | |
| B.157 | 2-Furyl | $4-Cl-C_6H_4$ | N | |
| B.158 | 2-Furyl | $2,4-Cl_2-C_6H_3$ | N | |
| B.159 | 2-Furyl | $2-Br-C_6H_4$ | N | |
| B.160 | 2-Furyl | $4-Br-C_6H_4$ | N | |
| B.161 | 2-Furyl | $2-F-C_6H_4$ | N | |
| B.162 | 2-Furyl | $4-F-C_6H_4$ | N | |
| B.163 | 2-Furyl | $2-CH_3-C_6H_4$ | N | |
| B.164 | 2-Furyl | $2-CH_3-C_6H_4$ | CH | |

Tabelle B (Fortsetzung)

| Nr. | A | B | Z | Fp. [°C]/IR [$cm^{-1}$] |
|---|---|---|---|---|
| B.165 | 2-Furyl | $2-CF_3-C_6H_4$ | N | |
| B.166 | 2-Furyl | $2-CF_3-C_6H_4$ | CH | |
| B.167 | 2-Furyl | $4-CF_3-C_6H_4$ | N | |
| B.168 | 2-Furyl | $2-OCH_3-C_6H_4$ | N | |
| B.169 | 2-Furyl | 4-Biphenyl | N | |
| B.170 | 2-Furyl | 2-Thienyl | N | |
| B.171 | 2-Furyl | 3-Thienyl | N | |
| B.172 | 2-Furyl | 2-Furyl | N | |
| B.173 | 2-Furyl | 4-Oxazolyl | N | |
| B.174 | 2-Furyl | 3-Pyridyl | N | |
| B.175 | 2-Furyl | 4-Thiazolyl | N | |
| B.176 | 3-Pyridyl | $C_6H_5$ | N | |
| B.177 | 3-Pyridyl | $2-Cl-C_6H_4$ | N | |
| B.178 | 3-Pyridyl | $4-Cl-C_6H_4$ | N | |
| B.179 | 3-Pyridyl | $2,4-Cl_2-C_6H_3$ | N | |
| B.180 | 3-Pyridyl | $2-F-C_6H_4$ | N | |
| B.181 | 3-Pyridyl | $4-F-C_6H_4$ | N | |
| B.182 | 3-Pyridyl | $2-CF_3-C_6H_4$ | N | |
| B.183 | 3-Pyridyl | $2-CH_3-C_6H_4$ | N | |
| B.184 | 3-Pyridyl | $2-Br-C_6H_4$ | N | |
| B.185 | 4-Pyridyl | $C_6H_5$ | N | |
| B.186 | 4-Pyridyl | $2-Cl-C_6H_4$ | N | |
| B.187 | 4-Pyridyl | $4-Cl-C_6H_4$ | N | |
| B.188 | 4-Pyridyl | $2,4-Cl_2-C_6H_3$ | N | |

Tabelle B (Fortsetzung)

| Nr. | A | B | Z | Fp. [°C]/IR [$cm^{-1}$] |
|---|---|---|---|---|
| B.189 | 4-Pyridyl | 2-F-$C_6H_4$ | N | |
| B.190 | 4-Pyridyl | 4-F-$C_6H_4$ | N | |
| B.191 | 4-Pyridyl | 2-$CF_3$-$C_6H_4$ | N | |
| B.192 | 4-Pyridyl | 2-$CH_3$-$C_6H_4$ | N | |
| B.193 | 4-Pyridyl | 2-Br-$C_6H_4$ | N | |
| B.194 | 4-Pyridyl | 2-Thienyl | N | |
| B.195 | 4-Pyridyl | 3-Thienyl | N | |
| B.196 | 4-Pyridyl | 2-Furyl | N | |
| B.197 | 4-Pyridyl | 3-Pyridyl | N | |
| B.198 | 2-Pyridyl | $C_6H_5$ | N | |
| B.199 | 2-Pyridyl | 2-Cl-$C_6H_4$ | N | |
| B.200 | 2-Pyridyl | 4-Cl-$C_6H_4$ | N | |
| B.201 | 2-Pyridyl | 2,4-$Cl_2$-$C_6H_3$ | N | |
| B.202 | 2-Pyridyl | 4-F-$C_6H_4$ | N | |
| B.203 | 2-Pyridyl | 2-$CF_3$-$C_6H_4$ | N | |
| B.204 | 2-Pyridyl | 2-$CH_3$-$C_6H_4$ | N | |
| B.205 | 4-Oxazolyl | $C_6H_5$ | N | |
| B.206 | 4-Oxazolyl | 2-Cl-$C_6H_4$ | N | |
| B.207 | 4-Oxazolyl | 2-$CF_3$-$C_6H_4$ | N | |
| B.208 | 4-Oxazolyl | 2-$CH_3$-$C_6H_4$ | N | |
| B.209 | 4-Thiazolyl | $C_6H_5$ | N | |
| B.210 | 4-Thiazolyl | 2-Cl-$C_6H_4$ | N | |
| B.211 | 4-Thiazolyl | 2-$CF_3$-$C_6H_4$ | N | |
| B.212 | 4-Thiazolyl | 2-$CH_3$-$C_6H_4$ | N | |

Tabelle B (Fortsetzung)

| Nr. | A | B | Z | Fp. [°C]/IR [$cm^{-1}$] |
|---|---|---|---|---|
| B.213 | 4-Thiazolyl | 2-Thienyl | N | |
| B.214 | 4-Thiazolyl | 2-Furyl | N | |
| B.215 | 4-Thiazolyl | 3-Pyridyl | N | |
| B.216 | 5-Oxazolyl | $C_6H_5$ | N | |
| B.217 | 5-Oxazolyl | 2-Cl-$C_6H_4$ | N | |
| B.218 | 5-Oxazolyl | 2-$CH_3$-$C_6H_4$ | N | |
| B.219 | 5-Oxazolyl | 3-Thienyl | N | |
| B.220 | 5-Thiazolyl | $C_6H_5$ | N | |
| B.221 | 5-Thiazolyl | 2-Cl-$C_6H_4$ | N | |
| B.222 | 5-Thiazolyl | 2-$CH_3$-$C_6H_4$ | N | |
| B.223 | 5-Thiazolyl | 2-Furyl | N | |
| B.224 | 3-Isoxazolyl | $C_6H_5$ | N | |
| B.225 | 3-Isoxazolyl | 2-Cl-$C_6H_4$ | N | |
| B.226 | 3-Isoxazolyl | 2-$CH_3$-$C_6H_4$ | N | |
| B.227 | 3-Isoxazolyl | 3-Thienyl | N | |
| B.228 | 4-Isoxazolyl | $C_6H_5$ | N | |
| B.229 | 4-Isoxazolyl | 2-Cl-$C_6H_4$ | N | |
| B.230 | 5-Isoxazolyl | $C_6H_5$ | N | |
| B.231 | 5-Isoxazolyl | 2-Cl-$C_6H_4$ | N | |
| B.232 | 4-Imidazolyl | $C_6H_5$ | N | |
| B.233 | 4-Imidazolyl | 2-Cl-$C_6H_4$ | N | |
| B.234 | Cyclohexyl | 2-Thienyl | N | |
| B.235 | Cyclohexyl | 3-Thienyl | N | |
| B.236 | Cyclohexyl | 2-Furyl | N | |

Tabelle B (Fortsetzung)

| Nr. | A | B | Z | Fp. [°C]/IR [$cm^{-1}$] |
|---|---|---|---|---|
| B.237 | Cyclohexyl | 2-Pyridyl | N | |
| B.238 | Cyclohexyl | 3-Pyridyl | N | |
| B.239 | Cyclohexyl | 4-Pyridyl | N | |
| B.240 | Cyclohexyl | 4-Thiazolyl | N | |
| B.241 | Cyclohexyl | 5-Oxazolyl | N | |
| B.242 | tert.-$C_4H_9$ | 2-Thienyl | N | |
| B.243 | tert.-$C_4H_9$ | 3-Thienyl | N | |
| B.244 | tert.-$C_4H_9$ | 2-Furyl | N | |
| B.245 | tert.-$C_4H_9$ | 2-Pyridyl | N | |
| B.246 | tert.-$C_4H_9$ | 3-Pyridyl | N | |
| B.247 | tert.-$C_4H_9$ | 4-Pyridyl | N | |
| B.248 | tert.-$C_4H_9$ | 4-Isoxazolyl | N | |
| B.249 | 4-F-$C_6H_4$ | 2-$CH_3$-$C_6H_4$ | N | 99-102 |
| B.250 | 4-F-$C_6H_4$ | 2-$CH_3$-$C_6H_4$ | CH | 1606,1512,1225, 1193,845,748 |
| B.251 | 3-F-$C_6H_4$ | 2-$CH_3$-$C_6H_4$ | N | |
| B.252 | 3-F-$C_6H_4$ | 2-$CH_3$-$C_6H_4$ | CH | |
| B.253 | 2-F-$C_6H_4$ | 2-$CH_3$-$C_6H_4$ | N | |
| B.254 | 2-F-$C_6H_4$ | 2-$CH_3$-$C_6H_4$ | CH | |
| B.255 | 2,4-$F_2$-$C_6H_3$ | 2-$CH_3$-$C_6H_4$ | N | |
| B.256 | 2,4-$F_2$-$C_6H_3$ | 2-$CH_3$-$C_6H_4$ | CH | |
| B.257 | 2-Cl-$C_6H_4$ | 2-$CH_3$-$C_6H_4$ | N | |
| B.258 | 2-Cl-$C_6H_4$ | 2-$CH_3$-$C_6H_4$ | CH | |
| B.259 | 3-Cl-$C_6H_4$ | 2-$CH_3$-$C_6H_4$ | N | |
| B.260 | 3-Cl-$C_6H_4$ | 2-$CH_3$-$C_6H_4$ | CH | |

Tabelle B (Fortsetzung)

| Nr. | A | B | Z | Fp. [°C]/IR [$cm^{-1}$] |
|---|---|---|---|---|
| B.261 | $4-Cl-C_6H_4$ | $2-CH_3-C_6H_4$ | N | 105-108 |
| B.262 | $4-Cl-C_6H_4$ | $2-CH_3-C_6H_4$ | CH | 99-102 |
| B.263 | $2,3-Cl_2-C_6H_3$ | $2-CH_3-C_6H_4$ | N | |
| B.264 | $2,3-Cl_2-C_6H_3$ | $2-CH_3-C_6H_4$ | CH | |
| B.265 | $2,4-Cl_2-C_6H_3$ | $2-CH_3-C_6H_4$ | N | |
| B.266 | $2,4-Cl_2-C_6H_3$ | $2-CH_3-C_6H_4$ | CH | |
| B.267 | $2-Br-C_6H_4$ | $2-CH_3-C_6H_4$ | N | |
| B.268 | $2-Br-C_6H_4$ | $2-CH_3-C_6H_4$ | CH | |
| B.269 | $4-Br-C_6H_4$ | $2-CH_3-C_6H_4$ | N | |
| B.270 | $4-Br-C_6H_4$ | $2-CH_3-C_6H_4$ | CH | |
| B.271 | $2-Cl-4-F-C_6H_3$ | $2-CH_3-C_6H_4$ | N | |
| B.272 | $2-Cl-4-F-C_6H_3$ | $2-CH_3-C_6H_4$ | CH | |
| B.273 | $3-NO_2-C_6H_4$ | $2-CH_3-C_6H_4$ | N | |
| B.274 | $3-NO_2-C_6H_4$ | $2-CH_3-C_6H_4$ | CH | |
| B.275 | $4-NO_2-C_6H_4$ | $2-CH_3-C_6H_4$ | N | |
| B.276 | $4-NO_2-C_6H_4$ | $2-CH_3-C_6H_4$ | CH | |
| B.277 | $3-NH_2-C_6H_4$ | $2-CH_3-C_6H_4$ | N | |
| B.278 | $3-NH_2-C_6H_4$ | $2-CH_3-C_6H_4$ | CH | |
| B.279 | $2-CH_3-C_6H_4$ | $2-CH_3-C_6H_4$ | N | |
| B.280 | $2-CH_3-C_6H_4$ | $2-CH_3-C_6H_4$ | CH | |
| B.281 | $4-CH_3-C_6H_4$ | $2-CH_3-C_6H_4$ | N | |
| B.282 | $4-CH_3-C_6H_4$ | $2-CH_3-C_6H_4$ | CH | |
| B.283 | $2-CF_3-C_6H_4$ | $2-CH_3-C_6H_4$ | N | |
| B.284 | $2-CF_3-C_6H_4$ | $2-CH_3-C_6H_4$ | CH | |
| B.285 | $3-CF_3-C_6H_4$ | $2-CH_3-C_6H_4$ | N | |

Tabelle B (Fortsetzung)

| Nr. | A | B | Z | Fp. [°C]/IR [$cm^{-1}$] |
|---|---|---|---|---|
| B.286 | $3-CF_3-C_6H_4$ | $2-CH_3-C_6H_4$ | CH | |
| B.287 | $4-CF_3-C_6H_4$ | $2-CH_3-C_6H_4$ | N | |
| B.288 | $4-CF_3-C_6H_4$ | $2-CH_3-C_6H_4$ | CH | |
| B.289 | 4-tert.-$C_4H_9-C_6H_4$ | $2-CH_3-C_6H_4$ | N | |
| B.290 | 4-tert.-$C_4H_9-C_6H_4$ | $2-CH_3-C_6H_4$ | CH | |
| B.291 | $2-OCH_3-C_6H_4$ | $2-CH_3-C_6H_4$ | N | |
| B.292 | $2-OCH_3-C_6H_4$ | $2-CH_3-C_6H_4$ | CH | |
| B.293 | $3-OCH_3-C_6H_4$ | $2-CH_3-C_6H_4$ | N | |
| B.294 | $3-OCH_3-C_6H_4$ | $2-CH_3-C_6H_4$ | CH | |
| B.295 | $4-OCH_3-C_6H_4$ | $2-CH_3-C_6H_4$ | N | |
| B.296 | $4-OCH_3-C_6H_4$ | $2-CH_3-C_6H_4$ | CH | |
| B.297 | $4-C_4H_9O-C_6H_4$ | $2-CH_3-C_6H_4$ | N | |
| B.298 | $4-C_4H_9O-C_6H_4$ | $2-CH_3-C_6H_4$ | CH | |
| B.299 | $C_6H_5$ | $2-CH_3-C_6H_4$ | N | 1492, 1447, 1271, 1139, 758, 698 |
| B.300 | $C_6H_5$ | $2-CH_3-C_6H_4$ | CH | |
| B.301 | $4-C_6H_5-C_6H_4$ | $2-CH_3-C_6H_4$ | N | |
| B.302 | $4-C_6H_5-C_6H_4$ | $2-CH_3-C_6H_4$ | CH | |
| B.303 | 1-Naphthyl | $2-CH_3-C_6H_4$ | N | |
| B.304 | 1-Naphthyl | $2-CH_3-C_6H_4$ | CH | |
| B.305 | 2-Naphthyl | $2-CH_3-C_6H_4$ | N | |
| B.306 | 2-Naphthyl | $2-CH_3-C_6H_4$ | CH | |
| B.307 | $4-C_6H_5-O-C_6H_4$ | $2-CH_3-C_6H_4$ | N | |
| B.308 | $4-C_6H_5-O-C_6H_4$ | $2-CH_3-C_6H_4$ | CH | |
| B.309 | $CH_3$ | $2-CH_3-C_6H_4$ | N | |

Tabelle B (Fortsetzung)

| Nr. | A | B | Z | Fp. [°C]/IR [$cm^{-1}$] |
|---|---|---|---|---|
| B.310 | $CH_3$ | $2-CH_3-C_6H_4$ | CH | |
| B.311 | $C_2H_5$ | $2-CH_3-C_6H_4$ | N | |
| B.312 | $C_2H_5$ | $2-CH_3-C_6H_4$ | CH | |
| B.313 | tert.-$C_4H_9$ | $2-CH_3-C_6H_4$ | N | |
| B.314 | tert.-$C_4H_9$ | $2-CH_3-C_6H_4$ | CH | |
| B.315 | Cyclopropyl | $2-CH_3-C_6H_4$ | N | |
| B.316 | Cyclopropyl | $2-CH_3-C_6H_4$ | CH | |
| B.317 | Cyclopentyl | $2-CH_3-C_6H_4$ | N | |
| B.318 | Cyclopentyl | $2-CH_3-C_6H_4$ | CH | |
| B.319 | Cyclohexyl | $2-CH_3-C_6H_4$ | N | |
| B.320 | Cyclohexyl | $2-CH_3-C_6H_4$ | CH | |
| B.321 | 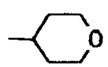 | $2-CH_3-C_6H_4$ | N | |
| B.322 | 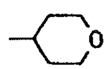 | $2-CH_3-C_6H_4$ | CH | |

Die Azolylmethyloxirane I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des Azolylmethyloxirans gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung wurden im allgemeinen Wirkstoffmengen von 0,001 bis 50 g, insbesondere 0,01 bis 10 g, je Kilogramm Saatgut verwendet.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. eine Lösung aus 90 Gew.-Teilen der Verbindung Nr. B.001 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. B.100, 80 Gew.Teilen Xylol, 10 Gew.-Teilen

des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. B.001, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. B.100, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. B.301, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. B.302 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. B.313, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. B.314, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. B.301, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums:

Anwendungsbeispiele

Als Vergleichssubstanzen dienten cis-2-(1,2,4-Triazol-1-yl-methyl)-2-(4-methylphenyl)-3-(2-fluorphenyl)-oxiran (V1) - bekannt aus EP 196 038 und cis-2-(1,2,4-Triazol-1-yl-methyl)-2-(4-chlorphenyl)-3-(4-tert.butylphenyl)-oxiran (V2) - bekannt aus EP 94 564:

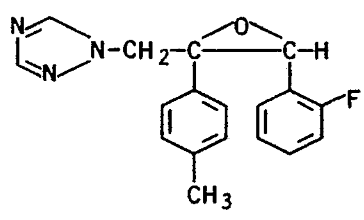

V1

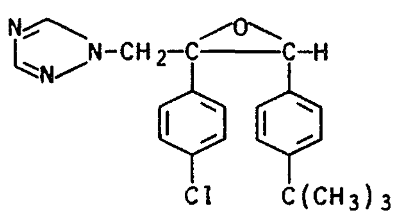

V2

Beispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22 °C in eine Kammer mit 90 bis 95 % relativer Luftfeuchtigkeit gestellt. Während dieser Zeit keimten die Sporen

aus und die Keimschläuche drangen in das Blattgewebe ein. Die infizierten Pflanzen wurden anschließend mit 0,006 gew.-%iger wäßriger Spritzbrühe, die 80 % (Gew. %) Wirkstoff und 20 % Emulgiermittel in der Trockensubstanz enthielten, tropfnaß gespritzt und nach dem Antrocknen des Spritzbelages im Gewächshaus bei einer Temperatur zwischen 20 und 22 °C und 65 bis 70 % relativer Luftfeuchtigkeit aufgestellt. Nach 8 Tagen beurteilte man das Ausmaß der Rostpilzentwicklung auf den Blättern.

Das Ergebnis zeigt, daß die Wirkstoffe B.249 und B.261 bei der Anwendung als 0,006 %ige (Gew.%) Spritzbrühe eine bessere fungizide Wirkung zeigen (100 %) als die bekannten Vergleichswirkstoffe V1 (80 %) und V2 (50 %).

Beispiel 2

Wirksamkeit gegen Weizenmehltau

Blätter von in Töpfen gewachsenen Weizenkeimlingen der Sorte "Kanzler" wurden mit 0,006 gew.-%iger wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe B.249 und B.261 bei der Anwendung als 0,006 %ige Spritzbrühe eine bessere fungizide Wirkung aufweisen (100 %) als die bekannten Vergleichswirkstoffe V1 (80 %) und V2 (65 %).

Beispiel 3

Wirksamkeit gegen Pyrenophora teres

Gerstenkeimlinge der Sorte "Igri" wurden im Zweiblattstadium mit 0,05 %igen wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres infiziert und für 48 Stunden in eine Klimakammer mit hoher Luftfeuchtigkeit bei 18 °C gestellt. Anschließend wurden die Pflanzen im Gewächshaus bei 20 bis 22 °C und 70 % relativer Luftfeuchtigkeit für weitere 5 Tage kultiviert. Danach wurde das Ausmaß des Pilzbefalls ermittelt.

Das Ergebnis zeigt, daß die Wirkstoffe B.249, B.250, B.261 und B.262 bei der Anwendung als 0,05 %ige Spritzbrühe eine bessere fungizide Wirkung aufweisen (100 %) als der bekannte Vergleichswirkstoff V1 (65 %).

## Ansprüche

1. Azolylmethyloxirane der allgemeinen Formel I

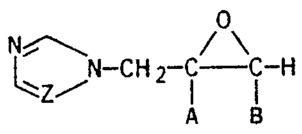

I

in der die Substituenten die folgende Bedeutung haben:

A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,

wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,

mit der Maßgabe, daß mindestens einer der Substituenten A oder B eine der oben definierten Heteroarylgruppen bedeutet, es sei denn, B bedeutet eine o-Methylphenylgruppe;
Z CH oder N
sowie ihre pflanzenverträglichen Säureadditionssalze und Metallkomplexe,
ausgenommen 2-(Imidazol-1-ylmethyl)-2-(4-fluorphenyl)-3-(pyrid-3-yl)-oxiran.

2. Azolylmethyloxirane der Formel I nach Anspruch 1, wobei A einen Phenylrest, der durch Fluor oder Chlor substituiert sein kann, und B einen o-Methylphenylrest bedeutet.

3. Azolylmethyloxirane der Formel I nach Anspruch 1, wobei A P-Fluorphenyl oder p-Chlorphenyl und B o-Methylphenyl bedeutet.

4. Verfahren zur Herstellung der Azolylmethyloxirane der Formel I

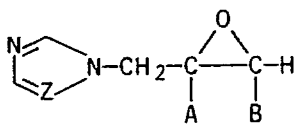

I,

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
ausgenommen 2-(Imidazol-1-ylmethyl)-2-(4-fluorphenyl)-3-(pyrid-3-yl)-oxiran, dadurch gekennzeichnet, daß man entweder
a) ein Azol der Formel IIa

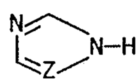

IIa

mit einem Oxiran der Formel III

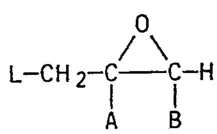

III,

wobei L eine nukleophile Abgangsgruppe bedeutet, umsetzt,
oder
b) ein Azolyl-Anion der Formel IIb

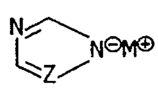

IIb

wobei M ein Alkalimetallatom bedeutet,
mit einem Oxiran der Formel III umsetzt,
oder
c) ein 3-Azolylprop-1-en der Formel IV

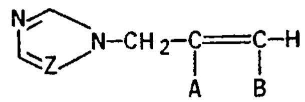

IV

epoxidiert,
und die erhaltenen Verbindungen gewünschtenfalls in ihre Salze oder Metallkomplexe überführt.

5. Fungizides Mittel, enthaltend eine fungizid wirksame Menge eines Azolylmethyloxirans der allgemeinen Formel I

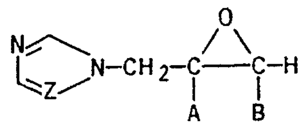

I,

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
ausgenommen 2-(Imidazol-1-ylmethyl)-2-(4-fluorphenyl)-3-(pyrid-3-yl)-oxiran, oder dessen pflanzenverträglichen Säureadditionssalzes oder dessen Metallkomplexes und einen flüssigen oder festen Trägerstoff.

6. Verfahren zur Bekämpfung von Pilzen, dadurch gekennzeichnet, daß man eine fungizid wirksame Menge eines Azolylmethyloxirans der allgemeinen Formel I

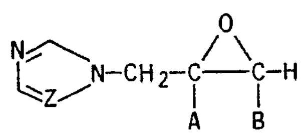

I,

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
ausgenommen 2-(Imidazol-1-ylmethyl)-2-(4-fluorphenyl)-3-(pyrid-3-yl)-oxiran, oder dessen pflanzenverträglichen Säureadditionssalzes oder Metallkomplexes, auf Pilze, vom Pilzbefall bedrohte Pflanzen, deren Lebensraum oder auf das Saatgut der bedrohten Pflanzen einwirken läßt.

7. Oxirane der allgemeinen Formel III

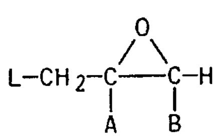

III

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyra-

nyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
L Halogen oder einen Rest R-$SO_2$-O- und R $C_1$-$C_6$-Alkyl, partiell oder vollständig halogeniertes $C_1$-$C_4$-Alkyl oder Phenyl, das durch Halogen, $C_1$-$C_4$-Alkyl oder Nitro substituiert sein kann.

Patentansprüche für folgende Vertragsstaaten: ES, GR

1. Verfahren zur Herstellung von Azolylmethyloxiranen der allgemeinen Formel I

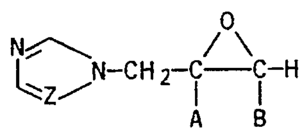

I

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
sowie ihre pflanzenverträglichen Säureadditionssalze und Metallkomplexe,
ausgenommen 2-(Imidazol-1-ylmethyl)-2-(4-fluorphenyl)-3-(pyrid-3-yl)-oxiran,
dadurch gekennzeichnet, daß man entweder
a) ein Azol der Formel IIa

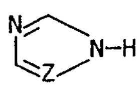

IIa

mit einem Oxiran der Formel III

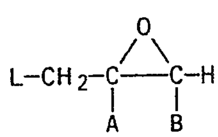

III,

wobei L eine nukleophile Abgangsgruppe bedeutet, umsetzt,
oder
b) ein Azolyl-Anion der Formel IIb

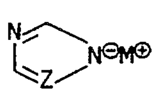

IIb

wobei M ein Alkalimetallatom bedeutet,
mit einem Oxiran der Formel III umsetzt,
oder

c) ein 3-Azolylprop-1-en der Formel IV

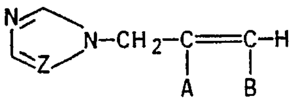

IV

epoxidiert,
und die erhaltenen Verbindungen gewünschtenfalls in ihre Salze oder Metallkomplexe überführt.
2. Verfahren nach Anspruch 1, dadurch gekennzeichnet, daß man es auf die Herstellung von Azolylmethyloxiranen I, wobei A einen Phenylrest, der durch Fluor oder Chlor substituiert sein kann, und B einen o-Methylphenylrest bedeutet, anwendet.
3. Verfahren nach Anspruch 1, dadurch gekennzeichnet, daß man es auf die Herstellung von Azolylmethyloxiranen I, wobei A p-Fluorphenyl oder p-Chlorphenyl und B o-Methylphenyl bedeutet, anwendet.
4. Fungizides Mittel, enthaltend eine fungizid wirksame Menge eines Azolylmethyloxirans der allgemeinen Formel I

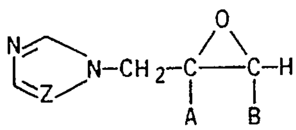

I

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
ausgenommen 2-(Imidazol-1-ylmethyl)-2-(4-fluorphenyl)-3-(pyrid-3-yl)-oxiran, oder dessen pflanzenverträglichen Säureadditionssalzes oder dessen Metallkomplexes und einen flüssigen oder festen Trägerstoff.
5. Verfahren zur Bekämpfung von Pilzen, dadurch gekennzeichnet, daß man eine fungizid wirksame Menge eines Azolylmethyloxirans der allgemeinen Formel I

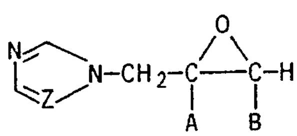

I,

in der die Substituenten die folgende Bedeutung haben:
A, B: 5- oder 6-gliedriges Heteroaryl, $C_1$-$C_8$-Alkyl, $C_3$-$C_8$-Cycloalkyl, Tetrahydropyranyl, Tetrahydrothiopyranyl, Phenyl, Biphenyl, Naphthyl oder Benzyl,
wobei diese Substituenten noch eine Nitro- oder Aminogruppe oder bis zu drei der folgenden Reste tragen können: Halogen, $C_1$-$C_4$-Alkyl, partiell oder vollständig halogeniertes $C_1$-$C_4$-Alkyl, $C_1$-$C_4$-Alkoxy oder Phenoxy,
mit der Maßgabe, daß mindestens einer der Substituenten A oder B eine der oben definierten Heteroarylgruppen bedeutet, es sei denn, B bedeutet eine o-Methylphenylgruppe;
Z CH oder N
ausgenommen 2-(Imidazol-1-ylmethyl)-2-(4-fluorphenyl)-3-(pyrid-3-yl)-oxiran, oder dessen pflanzenverträglichen Säureadditionssalzes oder Metallkomplexes, auf Pilze, vom Pilzbefall bedrohte Pflanzen, deren Lebensraum oder auf das Saatgut der bedrohten Pflanzen einwirken läßt.